# EUROPEAN PATENT SPECIFICATION

(11) **EP 1 699 921 B1**
(45) Date of publication and mention of the grant of the patent: **04.11.2009**
(21) Application number: 04700022.9
(22) Date of filing: 01.01.2004
(51) Int. Cl.: C12N 11/00, C12N 9/02, C12N 9/34

(54) **A METHOD FOR THE PREPARATION OF CROSS LINKED PROTEIN CRYSTALS**
METHODE ZUR HERSTELLUNG VON QUERVERNETZTEN PROTEIN KRISTALLEN
PROCEDE POUR L'ELABORATION DE CRISTAUX DE PROTEINES RETICULES

(43) Date of publication of application: 13.09.2006
(73) Proprietor: Council of Scientific and Industrial Research;an Indian Reg. body incorporated under Reg. of Societies Act (Act XXI of 1860), New Delhi 110 001 (IN)
(72) Inventor: ABRAHAM, Tholath, Emilia, Regional Research Lab., Trivandrum 695 019, Kerala (IN); BINDHU, Lekshmibai, V. A., Regional Research Lab., Trivandrum 695 019, Kerala (IN)
(74) Representative: Winter, Brandl, Fürniss, Hübner Röss, Kaiser, Polte Partnerschaft Patent- und Rechtsanwaltskanzlei
(86) International application number: PCT/IN2004/000002
(87) International publication number: WO 2005/066341

(56) References cited:
- WO-A-92/02617
- WO-A-99/12959
- US-A- 5 932 212
- MARGOLIN A L: "Novel crystalline catalysts" TRENDS IN BIOTECHNOLOGY, ELSEVIER PUBLICATIONS, CAMBRIDGE, GB, vol. 14, no. 7, 1 July 1996 (1996-07-01), pages 223-230, XP004035759 ISSN: 0167-7799
- ABRAHAM T E ET AL: "Crosslinked enzyme crystals of glucoamylase as a potent catalyst for biotransformations" CARBOHYDRATE RESEARCH, ELSEVIER SCIENTIFIC PUBLISHING COMPANY. AMSTERDAM, NL, vol. 339, no. 6, 28 April 2004 (2004-04-28), pages 1099-1104, XP004500589 ISSN: 0008-6215
- MARGOLIN, ALEXEY L. ET AL: "Protein crystals as novel catalytic materials" ANGEWANDTE CHEMIE, INTERNATIONAL EDITION , 40 (12), 2204 -2222 CODEN: ACIEF5; ISSN: 1433-7851, 2001, XP002296964
- GOVARDHAN CHANDRIKA P: "Crosslinking of enzymes for improved stability and performance" CURRENT OPINION IN BIOTECHNOLOGY, vol. 10, no. 4, August 1999 (1999-08), pages 331-335, XP002296965 ISSN: 0958-1669

## Description

The present invention relates to the method for the preparation of cross linked amylase or peroxidase crystals.

The main utility of the invention is to make stable cross linked amylase or peroxidase crystals, which can be used as a catalyst in the chemical reactions especially in organic solvents. In the method of the present invention by which enzyme crystals are produced, small protein crystals (crystals of approximately 100 microns in size) are grown from aqueous solutions, or aqueous solutions containing organic solvents, in which the enzyme catalysts is structurally and functionally stable. In a preferred embodiment, crystals are then crosslinked with a bifunctional reagent, such as glutaraldehyde. This crosslinking results in the stabilization of the crystal lattice contacts between the individual enzyme catalyst molecules constituting the crystal. As a result of this added stabilization, the crosslinked immobilized enzyme crystals can function at elevated temperatures, extremes of pH and in aqueous, organic, or near-anhydrous media, including mixtures of these. That is, a CLEC of the present invention can function in environments incompatible with the functional integrity of the corresponding uncrystallized, uncrosslinked, native enzyme or conventionally immobilized enzyme catalysts. In addition, CLECs made by this method can be subjected to lyophilization, producing a lyophilized CLEC which is resistant to protease degradation and can be stored in this lyophilized form at non-refrigerated (room) temperatures for extended periods of time, and which can be easily reconstituted in aqueous, organic, or mixed aqueous-organic solvents of choice, without the formation of amorphous suspensions and with minimal risk of denaturation.

The use of proteins, such as enzymes, as catalysts in industrial scale synthesis of specialty chemical and pharmaceuticals has received much attention [Dordick J.S, "Designing enzymes for use in organic, solvents" Biotechnol. progress (1992),8,259-267] Enzymes are recognized as useful tools for accomplishing chemical reactions in a stereo-, regio- and chemoselective manner. The ability of enzymes to function under mild conditions; ease of disposal and minimal waste production are further advantages associated with their use. Enzymes are also used for catalysis in organic solvents to solubilize substrates and products and to manipulate reaction kinetics and equilibrium in order to increase product yield. While enzymes offer impressive synthetic potential over current non enzymatic technology, their commercial use has been limited by disadvantages such as poor stability, variability in performance, difficulty of isolation and purification, difficulty in handling, high cost and long reaction times.

Furthermore, organic solvents are often incompatible with enzymes, leading to enzyme degradation or inactivation [A. M. Klibanov, "Asymmetric transformations Catalyzed by Enzymes in Organic Solvents", Acc. Chem. Res., 23, pp. 114-20 (1990]. In order for enzymes to function as viable industrial catalysts, they must be able to function without excessive intervention in the practical environments of manufacturing processes. Such environments include polar and non-polar organic solvents and aqueous-organic solvent mixtures. The low activity of enzymes and their aversion to organic solvents have remained barriers to widespread use of these proteins in routine organic syntheses. Even when such syntheses are catalyzed by enzymes, it is not unusual to see processes employing more enzyme than substrate by weight [Y. -F. Wang et al., J. Am. Chem. Soc., 110, pp. 7200-05 (1988)

Two methods designed to overcome these disadvantages--enzyme purification and enzyme immobilization--have addressed some of these disadvantages. However, they have not solved the problem of loss of enzyme activity or stability in organic solvents. Immobilization has actually exacerbated these problems by incurring higher costs and diluting the activity of the enzyme by the addition of support materials. Enzyme purification also incurs added cost and, in most cases fails to increase enzyme activity in organic solvents. Recent studies have demonstrated that enzyme activity in organic solvents is intimately related to water content, size and morphology of the catalyst particles and the enzyme microenvironment [A.M. Klibanov, "Enzymatic Catalysis in Anhydrous Organic Solvents", Trends in Biochem. Sci., 14, pp. 141-44 (1989)]. These parameters have been adjusted by preparing lyophilized complexes of enzymes with carbohydrates, organic buffers or salts [K. Dabulis and A. M. Klibanov, , "Dramatic Enhancement of Enzymatic Activity in Organic Solvents by Lyoprotectants", Biotechnol. Bioeng., 41, pp. 566-71 (1993)] However, despite the widespread use of lyophilization for preparation of enzymes for catalysis in organic solvents, in some instances, it may cause significant reversible denaturation of enzymes.

Other approaches to the problem of low enzyme activity in biotransformations involving organic solvents have included the use of surfactants. Surfactants have been mixed with an aqueous solution of an enzyme, the mixture dewatered and the resulting enzyme preparation used as a catalyst said to have enhanced activity in organic solvents. Surfactants or lipids have also been used to coat enzymes in order to solubilize them in organic solvents and, thus, increase chemical reaction rates [N. Kamiya et al., "Preparation of surfactant coated lipases utilizing the molecular imprinting technique" J.Fer.& Bioeng.(1996) 85(2)237-239]. After this procedure, the enzymes become soluble in organic solvents. Enzyme complexes soluble in organics are also described in V. M. Paradkar and J. S. Dordick, J. Am. Chem. Soc., 116, pp. 5009-10 (1994) (proteases) and Y. Okahata et al., J. Org. Chem., 60, pp. 2240-50(1995)(lipases).

The advent of crosslinked enzyme crystal (CLEC) technology provided a unique approach to solving the above-described disadvantages [N. L. St. Clair and M. A. Navia, J. Am. Chem.Soc., 114, pp. 7314-16 (1992)]. Crosslinked enzyme crystals retain their activity in environments that are normally incompatible with enzyme (soluble or immobilized) function. Such environments include prolonged exposure to high temperature and extreme pH. Additionally, in organic solvents and aqueous-organic solvent mixtures, crosslinked enzyme crystals exhibit both stability and activity far beyond that of their soluble or conventionally-immobilized counterparts. Since so many biocatalysis processes depend on stability and activity of an enzyme under sub-optimal conditions, crosslinked enzyme crystals are advantageously used in industrial, clinical and research settings. Thus, crosslinked enzyme crystals represent an important advance in the area of biocatalysis, as attractive and broadly applicable catalysts for organic synthesis reactions [R. A. Persichetti et al., "Cross-Linked Enzyme Crystals (CLECs) of Thermolysin in the Synthesis of Peptides", J. Am. Chem. Soc., 117, pp. 2732-37 (1995) and J. J. Lalonde et al., Chem tech(1997)38-45 Khalaf, N, J. Am. Chem. Society (1996) 118,5494-5495, Y.F.Wang, J.Org.Chem, (1997), 62(11) 3488-3495 -subtilisin ; J.Am.Chem.Soc.(1995) 117(26) 6845-6852.-lipase]. Despite the progress of protein catalysis technology in general, the need still exists for catalysts, which have high activity in organic solvents.

Reference may be made to a few potent commercialized cross linked proteins like CLEC.TM of Vertex Pharmaceuticals Inc and CLEC of Althus Biologicals, Inc. Cambridge,MA, for thermolysin, elastase, esterase, lipase, lysozyme, asparaginase, urease, nitrilase, hydantoinase, and protease[US Patent No: 5,849,296,Dec 1998,Crosslinked protein crystals, Navia *et.al ;* Canadian patent No: 2156177 and US Patent No.: 6,042,824 , March 2000, and US Patent No: 5,932,212, August 1999]. There is not much work carried out with enzymes that are glycoproteins, like amylases -amyloglucosidase which is an important enyme in starch hydrolysis and peroxidase enzymes are heme proteins very versatile catalysts which is used for novel polymer synthesis and environmental remediation. Glycoproteins are in general very difficult to crystalize . Amyloglucosidase enzmes are mainly used in large scale conversion of starchy materials to glucose. The depolymerisation of starch from the non-reducing end by this enzyme is a slow process and takes 8-20 hours depending on the concentration. The glucoamylase (amyloglucosidase) has a low temperature stability and above 60 degree C it denatures. The liquefaction step before this saccharification step is carried out at 90-110 degree C and takes place faster (15-60 min.) and in order to have a.faster process it is essential to have a depolymerising step also at a higher temperature. Attempts to improve the thermal stability by genetic manipulation and immobilization did not significantly improve the thermostability beyond 70 degree C. Where as the Cross linked crystal of AMG can be used at 80-90 Degree centigrade which in turn will shorten the hydrolysis time substantially.

Peroxidase enzymes which are mainly obtained from microorganisms (lignin peroxidase, Chloroperoxidase), Plants (Horse Radish peroxidase, Saccharum peroxidase and Ipomea peroxidase) get denatured during the polymerisation reactions in aqueous and organic phase. Cross linked crystals of these enzymes are sturdy and can withstand harsh conditions.

The main object of the present invention is to provide a method for the preparation of stable cross linked amylase or peroxidase crystals, which obviates the drawbacks as detailed above.

Another object of the present invention is to provide a method of immobilizing a protein, particularly an enzyme, by forming crystals of the enzyme and, generally, also crosslinking the resulting crystals through use of a bifunctional reagent.

Still another object of the present invention is that drying stable crosslinked amylase or peroxidase crystals in the presence of a surfactant and an organic solvent produces the stable crosslinked protein crystal formulations.

Yet another object of the present invention is that lyophilizing stable crosslinked amylase or peroxidase crystals in the presence of a surfactant and an organic solvent produces stable crosslinked protein crystal formulations that can be used as a means of improving the storage, handling, and manipulation of the properties of immobilized enzymes stored at room temperature.

Yet another object of the present invention is to make a desired product by means of a reaction catalyzed by a CLEC or a set of CLECs; and enhanced resistance to degradation (e.g., enhanced, protease resistance), and higher temperature-stability, relative to that of the native enzyme

Yet another object of the present invention is to provide stable crosslinked amylase or peroxidase crystal formulations which exhibit high activity and productivity as catalysts in chemical reactions involving organic solvents or aqueous-organic solvent mixtures. This level of activity and productivity is greater than that of soluble or conventionally immobilized proteins.

Yet another object of the present invention is to provide methods for producing stable crosslinked amylase or peroxidase crystal formulations and methods using them to optimize chemical reactions in organic solvents, including those used in industrial scale biocatalysis.

Accordingly the present invention provides a method for the preparation of stable cross linked amylase or peroxidase crystals,' wherein the amylase is a glucoamylase and the peroxidase is a plant peroxidase. The enzyme is first crystallized with a suitable salt and then immobilized by crosslinking the crystals of the protein with a multifunctional crosslinking agent, and the crosslinked protein crystals is lyophilized with a suitable surfactant for storage. The plant peroxidases preferably are horse radish Peroxidase and leaf peroxidases like Ipomea and Saccharum. The crosslinked enzyme crystals have a higher temperature stability and retain at least 90% activity after incubation for three hours in the presence of a concentration of protease that causes the soluble uncrosslinked form of the enzyme to lose at least 92% of its initial activity under the same conditions and the enzyme crystals that are crosslinked may be microcrystals having a cross-section of 10-100 microns and the crosslinked enzyme crystals may be used in an aqueous or organic medium for biotransformations, in an assay, diagnostic kit or biosensor for detecting an analyte, in an extracorporeal device for altering a component of a fluid, in producing a product such as using crosslinked Peroxidase crystals to produce novel chemicals and intermediates, separating a substance from a mixture, and in therapy.

In order that the invention herein described may be more fully understood, the following detailed description is set forth. In the description, the following terms are employed:
Organic Solvent--any solvent of non-aqueous origin.

Aqueous-Organic Solvent Mixture--a mixture comprising n % organic solvent, where n is between 1 and 99 and m % aqueous, where m is 100-n. Mixture Of Organic Solvents--a combination of at least two different organic solvents in any proportion.

Crosslinked Protein Crystal Formulation-a mixture of crosslinked protein crystals with one or more additional excipients, such as surfactants, salts, buffers, carbohydrates or polymers, in a dried, free-flowing powder or lyophilized form, rather than a slurry.

Catalytically Effective Amount--an amount of a crosslinked protein crystal formulation of this invention which is effective to protect, polymerise, hydrolyse, repair, or detoxify the area to which it is applied over some period of time.

The crosslinked protein crystal formulations of this invention are particularly advantageous because they retain high activity in harsh solvent environments that are typical of many industrial-scale chemical synthesis procedures. As a result of their crystalline nature, the crosslinked protein crystal components of these formulations achieve uniformity across the entire crosslinked crystal volume.

The intermolecular contacts and chemical crosslinks between the protein molecules constituting the crystal lattice maintain this uniformity, even when exchanged in organic or mixed aqueous-organic solvents. Even in such solvents, the protein molecules maintain a uniform distance from each other, forming well-defined stable pores within the crosslinked protein crystal formulations that facilitate access of substrate to the catalyst, as well as removal of product. In these crosslinked protein crystals, the lattice interactions, when fixed by chemical crosslinks, are particularly important in preventing denaturation, especially in organic solvents or mixed aqueous-organic solvents. Crosslinked protein crystals and the constituent proteins within the crystal lattice remain monodisperse in organic solvents, thus avoiding the problem of aggregation. These features of the crosslinked protein crystal components of crosslinked protein crystal formulations of this invention contribute to the high level of activity of those formulations in organic and aqueous-organic solvents.

In addition to their activity in organic solvents and aqueous-organic solvents, crosslinked protein crystal formulations according to this invention are particularly resistant to proteolysis, extremes of temperature and extremes of pH. The activity per crosslinked protein crystal unit volume is significantly higher than that of conventionally immobilized proteins or concentrated soluble proteins. This is because protein concentrations within the crosslinked protein crystal components of the formulations are close to theoretical limits.

By virtue of these advantages, the crosslinked protein crystal formulations of the present invention permit a maj or improvement in reaction efficiency. They provide improved yields under harsh conditions or situations requiring high throughput, enabling process chemists to concentrate on maximizing yield with less concern about reaction conditions.

The protein constituent of the crosslinked protein crystal formulations of this invention is a glucoamylase or a plant peroxidase. According to one embodiment of this invention, crosslinked protein crystal formulations are characterized by activity in either an organic solvent or an aqueous-organic solvent mixture, which is at least about 1.5 times greater than the activity of the equivalent amount of said protein in either crude form or pure form. In an alternate embodiment of this invention the activity level of such formulations ranges between about 1.5 times and about 10 times greater than the activity of the equivalent amount of said protein in either crude form or pure form.

Accordingly, the present invention provides a process for the preparation or stable cross linked amylase or peroxidase crystals, wherein the amylase is a glucoamylase and the peroxidase is a plant peroxidase, the process comprising the steps of:
(a) crystallizing the amylase or peroxidase enzymes in an aqueous solution containing a suitable salt in the presence of an organic cosolvent at a temperature ranging between 4° and 10°C for a period ranging between 5 hours to 20 days to obtain enzyme crystals having a particle size ranging between 50 to 150 microns,
(b) reacting the enzyme crystals obtained in step (a) with a multifunctional crosslinking agent in the presence of buffer of a pH ranging between 3-10 at a temperature ranging between 4° to 10°C to get the crossed linked enzyme crystals, wherein said cross-linking agent is selected from the group consisting of glutaraldehyde and starch dialdehyde;
(c) washing the cross linked crystals with a reagent that is capable of removing the excess of said multifunctional cross-linking agent so as to obtain the washed cross-linked enzyme; and
(d) coating the cross linked enzyme crystals with a suitable surfactant selected from the group consisting of cetyl trimethyl ammonium bromide, dioctyl sulfosuccinate, nonyl phenol ethoxylate, sorbitan trioleate, Tween 20, Tween 80 and Triton X-100 and lyophilizing it to obtain the stable product.

In one embodiment of the present invention said plant peroxidase is selected from the group consisting of Horseradish, Ipomea or Saccharum peroxidases.

In an another embodiment of the present invention said crystal is a microcrystal having a cross-section of 100 microns or less.

The cross linking reagent used in the process of the invention is glutaraldehyde or starch dialdehyde. The concentration of cross linking agent can be 1 to 50 mg per gram of the enzyme crystal.

The surfactant used in the process of the invention can be anionic, neutral, or cataionic and is selected from the group consisting of cetyl trimethyl ammonium bromide (CTAB), dioctyl sulfosuccinate (AOT), nonyl phenol ethoxylate, sorbitan trioleate, polyoxyethylenesorbitan monolaurate (TWEEN 20), TWEEN 80, and TRITON X-100.

In another embodiment of the invention said surfactant provides a weight ratio of cross-linked enzyme crystals to surfactant between about 1:1 and about 1:5, preferably between about 1:1 and about 1:2.

In an another embodiment of the invention coating with the surfactant is carried out by contacting the crosslinked enzyme crystals with surfactant for a period of time between about 5 minutes to 24 hours, preferably between about 30 minutes to 24 hours.

In an another embodiment of the invention wherein the said buffer used for the CLEC preparation can be 10 to 100 mM of standard acetate, phosphate, citrate or any suitable buffer with a pH in the range of 3-10. In another embodiment of the present invention the obtained Lyophilized cross linked enzyme crystal is having resistance to exogenous proteolysis, such that said crosslinked enzyme crystal retains at least 91% of its initial activity after incubation for three hours in the presence of a concentration of Protease that causes the soluble uncross-linked form of the enzyme that is crystallized to form said enzyme crystal that is crosslinked to lose at least 94% of its initial activity under the same conditions, wherein said crystal is in lyophilized form.

In an another embodiment of the invention the cross linked protein crystal permits said enzyme to act upon the substrate, thereby producing said product in said organic solvent or aqueous-organic solvent mixture.

In another embodiment of the invention said organic co solvent used is selected from the group consisting of octanes, diols, polyols, polyethers and water soluble polymers.

In another embodiment of the invention the organic cosolvent used is selected from the group consisting of toluene, octane, tetrahydrofuran, acetone, pyridine, diethylene glycol, 2-methyl-2,4-pentanediol, poly(ethylene glycol), triethylene glycol, 1,4-butanediol, 1,2-butanediol, 2,3,-dimethyl-2,3-butanediol, 1,2-butanediol, dimethyl tartrate, monoalkyl ethers of poly(ethylene glycol), dialkyl ethers of poly(ethylene glycol), and polyvinylpyrrolidone.

In another embodiment of the invention the cross linked protein crystal formulation comprises about 10 wt % and about 70 wt % of surfactant, by weight of the final formulation, preferably between about 25 wt % and about 45 wt % of surfactant, by weight of the final formulation.

In another embodiment of the invention the crystals may be used in an aqueous or organic medium for biotransformations, in an assay, diagnostic kit or biosensor for detecting an analyte, in producing a product such as using crosslinked Peroxidase crystals to produce novel polysaccharides, in separating a substance from a mixture, in therapy and in bioremediation of toxic effluents.

The present invention further provides a process for the preparation of glucose by hydrolysis of starch or maltodextrin, said' process comprising continuous saccharification of starch and/or maltodextrin by reacting a starch and /or maltodextrin solution in a packed bed reactor with cross-linked glucoamylase enzyme crystals.

The present invention further provides a process for the preparation of 2,4-dimethyl phenol dimer from monomer, said process comprising reacting 2,4-dimethyl phenol monomer with Hoseradish peroxidase enzyme crystals in the presence of hydrogen peroxide.

This invention also includes crosslinked protein crystal formulations characterized by a specific activity per milligram of solid in an organic solvent or an aqueous-organic solvent mixture which is at least about 2 times greater than that of said protein in either crude form or pure form. Crosslinked protein crystal formulations according to this invention may also be characterized by a specific activity per milligram of solid in an organic solvent or an aqueous-organic solvent mixture which is between about 2 times and about 50 times greater than that said protein in either crude form or pure form. And crosslinked protein crystal formulations of this invention may also be characterized by a specific activity per milligram of solid in an organic solvent or an aqueous-organic solvent mixture which has a level of activity greater than that of said protein in either crude form or pure form that is selected from the group consisting of at least about 10-50 times greater activity.

In another embodiment of this invention, crosslinked protein crystal formulations may also be characterized by activity in an organic solvent or an aqueous-organic solvent mixture, which is between about 2 times and about 10 times greater than the activity of crosslinked protein crystals which contain no surfactant.

In all of the crosslinked protein formulations described above, the stated activity levels may be exhibited in either organic solvents, or aqueous-organic solvents, or in both solvents. Such activity levels characterize all types of crosslinked protein crystal formulations, including crosslinked enzyme crystal formulations.

The crosslinked protein crystal formulations of this invention may be used in any of a number of chemical processes. Such processes include Industrial and research-scale processes such as organic synthesis of specialty chemicals and pharmaceuticals, synthesis of intermediates for the production of such products, chiral synthesis and resolution for optically pure pharmaceutical and specialty chemicals. Enzymatic conversion processes include oxidations, reductions, additions, hydrolysis, polymerization, and asymmetric conversions, including steroselective, stereospecific and regioselective reactions. Products, which may be produced using these reactions, include chiral organic molecules, peptides, polymers, carbohydrates, lipids and other chemical species.

In carrying out any of the above-enumerated reactions, it will be understood by those of skill in the art that the organic solvent or aqueous-organic solvent chosen for the particular reaction should be one which is compatible with the protein constituent of the crosslinked protein crystal, as well as the surfactant used to stabilize the crosslinked protein crystal. Organic solvents may be selected from the group consisting of diols, polyols, polyethers, water-soluble polymers and mixtures thereof. Examples of organic solvents include toluene, octane, tetrahydrofuran, hexane, DMSO, acetone, and pyridine. Further examples include hydrophobic or polar organic solvents such as, water miscible or water imiscible solvents, diethylene glycol, 2-methyl-2,4-pentanediol, poly(ethylene glycol), triethylene glycol, 1,4-butanediol, 1,2-butanediol, 2,3,-dimethyl-2, 3-butanediol, 1,2-butanediol, dimethyl tartrate, monoalkyl ethers of poly(ethylene glycol), dialkyl ethers of poly(ethylene glycol), and polyvinylpyrrolidone, or mixtures thereof.

According to one embodiment, this invention includes methods for producing a selected product in an organic solvent or an aqueous-organic solvent mixture by combining at least one substrate and at least one protein which acts upon the substrate in the presence of an organic solvent or an aqueous-organic solvent mixture--said protein being a crosslinked protein crystal formulation-and maintaining the combination under conditions, which permit, said protein to act upon the substrate, thereby producing the selected product. Products which may be produced in such methods include, for example, chiral organic molecules, peptides, carbohydrates, polymers and lipids.

According to one embodiment of this invention, crosslinked protein crystal formulations may be used as a component of a biosensor for detecting an analyte of interest in a sample, for example, a fluid. Such a biosensor comprises (a) a crosslinked protein crystal formulation, wherein said protein has the activity of acting on the analyte of interest or on a reactant in a reaction in which the analyte of interest participates; (b) a retaining means for said crosslinked protein crystal formulation, said retaining means comprising a material which allows contact between said crosslinked protein crystal formulation and a sample, said sample containing either (1) the analyte upon which the protein acts or (2) a reactant in a reaction in which the analyte participates; and, optionally,(c) a signal transducer which produces a signal in the presence or absence of the analyte. The means for detecting the activity of the protein on the analyte or reactant may be selected from the group consisting of pH electrodes, light sensing devices, heat sensing devices and means for detecting electrical charges. The signal transducer may be selected from the group consisting of optical transducers, electrical transducers, electromagnetic transducers and chemical transducers.

Thus, crosslinked protein crystal formulations according to this invention may be advantageously used instead of conventional soluble or immobilized proteins in biosensors. Such use of the crosslinked protein crystal formulations of this invention provides biosensors characterized by higher degrees of sensitivity, volumeric productivity and throughput than those of biosensors based on conventional soluble or immobilized proteins.

Crosslinked protein crystal formulations according to this invention may also be used in various environmental applications. They may be used in place of conventional soluble or immobilized proteins for environmental purposes, such as removal of residual starch and colour from waste water from paper, leather, and distilleries, degradation of dye effluents from textile industries, removal of toxic compounds like phenolics and chlorinated phenolics from the environment.

This invention also includes methods for increasing the activity of crosslinked protein crystals in an organic solvent or an aqueous-organic solvent mixture comprising the steps of combining the crosslinked protein crystals with a surfactant to produce a combination and drying the combination of crosslinked protein crystals and surfactant in the presence of an organic solvent to form a crosslinked protein crystal formulation.

Alternatively, crosslinked protein crystal formulations according to this invention may be used as ingredients in topical creams and lotions, for skin protection or detoxification. They may also be used as anti-oxidants in cosmetics. They can also be used for oral cavity hygiene product formulations.

The crosslinked protein crystal formulations may be in a variety of conventional depot forms employed for topical administration to provide reactive topical compositions. These include, for example, semi-solid and liquid dosage forms, such as liquid solutions or suspensions, gels, creams, emulsions, lotions, slurries, powders, sprays, foams, pastes, ointments, salves, balms and drops.

### Preparation of Crosslinked Enzyme Crystal Formulation

**Enzyme Crystallization:** Enzyme crystals are grown by the controlled precipitation of enzyme out of aqueous solution or aqueous solution-containing organic solvents and salts. Conditions to be controlled include, for example, the rate of evaporation of solvent, concentration of salt, the presence of appropriate co-solutes and buffers, pH and temperature. A comprehensive review of the various factors affecting the crystallization of proteins has been published by McPherson, [Methods Enzymol., 114, pp. 112-20 (1985)] have compiled comprehensive lists of proteins and nucleic acids that have been crystallized, as well as the conditions under which they were crystallized.

Enzymes, crystallized to form the crosslinked enzyme crystal component of the formulations according to this invention are glucoamylase and plant peroxidases such as Horse radish peroxidase, Ipomea peroxidase and Saccharum peroxidase, lignin peroxidase.

For use in crosslinked enzyme crystal formulations according to this invention, the large single crystals that are needed for X-ray diffraction analysis are not required. Microcrystalline showers are suitable. In general, crystals are produced by combining the enzyme to be crystallized with an appropriate aqueous solvent or aqueous solvent containing appropriate precipitating agents, such as salts or organics. The solvent is combined with the protein at a temperature determined experimentally to be appropriate for the induction of crystallization and acceptable for the maintenance of enzyme activity and stability. The solvent can optionally include co-solutes, such as divalent cations, co-factors or chaotropes, as well as buffer species to control pH. The need for co-solutes and their concentrations are determined experimentally to facilitate crystallization. In an industrial scale process, the controlled precipitation leading to crystallization can best be carried out by the simple combination of protein, precipitant, co-solutes and, optionally, buffers in a batch process.

Alternative laboratory crystallization methods, such as dialysis or vapor diffusion can also be adapted. Occasionally, incompatibility between the crosslinking reagent and the crystallization medium might require exchanging the crystals into a more suitable solvent system.

### Crosslinking of Enzyme Crystals

Once enzyme crystals have been grown in a suitable medium they can be crosslinked. Crosslinking results in stabilization of the crystal lattice by introducing covalent links between the constituent enzyme molecules of the crystal. This makes possible the transfer of enzyme into an alternate reaction environment that might otherwise be incompatible with the existence of the crystal lattice or even with the existence of intact protein. Crosslinking can be achieved by a wide variety of multifunctional reagents, including bifunctional reagents. According to a preferred embodiment of this invention, the crosslinking agent is glutaraldehyde. Crosslinking with glutaraldehyde forms strong covalent bonds primarily between lysine amino acid residues within and between the enzyme molecules in the crystal lattice. The crosslinking interactions prevent the constituent enzyme molecules in the crystal from going back into solution, effectively insolubilizing or immobilizing the enzyme molecules into microcrystalline particles (preferably having lengths 10- 100 microns).

**Exposure of Crosslinked Enzyme Crystals to Surfactants** : Crosslinked enzyme crystals prepared as described above, may be used to prepare enzyme crystal formulations for reactions in organic solvents and aqueous-organic solvent mixtures by being contacted with a surfactant as described above. After exposure of the crosslinked enzyme crystals to the surfactant and subsequent drying in the presence of an organic solvent, the resulting crosslinked enzyme crystal formulation is particularly active and stable in organic solvents and aqueous-organic solvent mixtures. Surfactants useful to prepare crosslinked enzyme crystal formulations according to this invention include cationic, anionic, non-ionic or amphoteric, or mixtures thereof. The preferred surfactant will depend upon the particular enzyme component of the crosslinked enzyme crystals to be used to prepare the crosslinked enzyme crystal formulation. This may be determined by carrying out a routine screening procedure based on a reaction catalyzed by the particular enzyme.

Generally, in order to prepare crosslinked enzyme crystal formulations, the surfactant should be added to a crosslinked enzyme crystal-containing solution in an amount sufficient to allow the surfactant to equilibrate with and/or penetrate the crosslinked enzyme crystals. Such an amount is one which provides a weight ratio of crosslinked enzyme crystals to surfactant between about 1:1 and about 1:5, preferably between about 1:1 and about 1:2. The crosslinked enzyme crystals are contacted with surfactant for a period of time between about 5 minutes and about 24 hours, preferably between about 30 minutes and about 24 hours. Following that contact, the crosslinked enzyme crystal/surfactant combination may be dried in the presence of an organic solvent to form the crosslinked enzyme crystal formulation.

The choice co organic solvent and length of drying time will depend on the particular crosslinked enzyme crystals and the particular surfactant used to produce the crosslinked enzyme crystal formulations. Nevertheless, the solvent and drying time should be those which provide a crosslinked enzyme crystal formulation characterized by a water content that permits the formulation to have maximum activity and stability in organic solvents or aqueous-organic solvent mixtures. According to one embodiment of this invention, the drying time may be between about 5 minutes and about 24 hours, preferably between about 30 minutes and about 24 hours. The organic solvent used in the drying step may be present in an amount between about 10 wt % and about 90 wt % of the total mixture, preferably between about 40 wt % and about 80 wt % of the total mixture.

Alternatively, the crosslinked enzyme crystal/surfactant combination may be lyophilized in the presence of an organic solvent. Lyophilization may be carried out for a period of time between about 30 minutes and about 18 hours.

The resulting crosslinked enzyme crystal formulation should contain between about 10 wt % and about 70 wt % of surfactant, by weight of the final formulation, preferably between about 25 wt % and about 45 wt % of surfactant, by weight of the final formulation.

In order that this invention may be better understood, the following examples are set forth. These examples are for the purpose of illustration only and are not to be construed as limiting the scope of the invention in any matter.

### EXAMPLE 1

### Preparation of A Crosslinked AMG Crystal Formulation

A slurry of 250ml Glucoamylase (Amyloglucosidase E.C: 3.2.1.3,α-1,4- Glucan glucohydrolase) was obtained from NOVO (Denmark, from A.niger , 22.623 IU/ml&Sp.activity 0.085 IU/mg)) was taken and higher mol.wt protein impurities are removed by 25% ammmonium sulphate cut off. The purified Glucoamylase enzyme was taken in acetate buffer (0.5M, pH-4.5), Isopropyl alcohol(20%) and surfactant (1%) were added along with sufficient saturated ammonium sulphate solution to crystallise the enzyme. The solution was stirred at 4°C for 30 minutes and then kept for 16 hours at the same temperature. The crystals formed were seperated by centrifugation at 10,000 rpm at 4 degree C for 10 min. Excess ammonium sulphate was removed by dialysis and then the solution was lyophilized to obtain glucoamylase crystals. The crystals were suspended in minimum amount of acetate buffer (0.5M, pH-4.5) containing starch (1%) and/or Bovine serum albumin (1mg/ml) before cross linking with glutaraldehyde (50% glutaraldehyde in 4 volume 0.2M phosphate buffer of pH 7.0). After 2 hour, the crosslinking reaction was stopped by washing the crosslinked crystals extensively in a filter press with an approximately 1.5 crystal slurry volume of buffer (50 mM acetate buffer pH 4.5), 6M urea, and 6M NaCl.). The crystal yield was about 270 grams.

A 30 gram aliquot of the above-prepared crosslinked AMG enzyme crystals was suspended in 340 ml storage buffer (0.05M acetate buffer, pH 4.5) and the mixture poured into a sintered glass funnel (porosity. about.10-20.mu.) at room temperature. The enzyme crystals were exposed to the surfactant AOT. This surfactant was selected by the screening process.

The buffer above the crosslinked AMG crystals was filtered in a sintered glass funnel (described above), keeping the enzyme crystals wet throughout the process. The surfactant Tween-20 was added together with the solvent 2-butanone, such that the ratio of surfactant : crosslinked enzyme crystals was 1:1 (30 g surfactant:30 g glucoamylase =30 ml). This was done by pouring a mixture of 30 ml 2-butanone and 30 ml surfactant, for a total of 60 ml, on top of the crosslinked enzyme crystals. A gentle suction was applied to ensure that the crosslinked enzyme crystals were coated with the surfactant and so that the enzyme cake did not dry. After 30 minutes at room temperature, the mixture was then transferred to a drying vessel lyophilized to a water content of about 1-3%. The cross linked crystal obtained has a Sp.activity of 0.0687IU/mg and a yield of 50.66 % of the original activity of the enzyme under optimized conditions of preparation. The crystals are rhombohedral having a size around 100 microns and density of 1.8926 g/cm³ and a surface area of 0.7867 m²/g. The pH optimum of the glucoamylase crystals was 4.5 which is same as the soluble enzyme. The Michaelis's constant (Km) for the Crosslinked glucoamylase crystal was 4000 mg/ml where as for soluble enzyme Km was only 454.5mg/ml with starch as substrate at pH 4.5 and at 60°C.
**1. Density:** Density of the Glucoamylase CLEC was obtained using a Helium Auto Pycnometer(Micromeritics 1320).
**2. Surface area**: Surface area was obtained by BET apparatus using liquid nitrogen as the adsorbent.
**3. Crystal Structure**: The crystal structure was observed under Scanning electron microscope (GEOL, Japan) at 10KV accelerating voltage.
**4. Determination of Enzyme Activity**
   0.2 ml enzyme solution is added to 1 ml of 4% starch solution in 0.2 M acetate buffer (pH 4.5) and this is incubated at 60°C. for exactly 1h. After 1h, the enzyme reaction is terminated by adding 0.8ml of 4N NaOH. The dextrose formed is determined using the Lane-Eynon method. Activity of glucoamylase CLEC is determined by the same procedure. Instead of adding the enzyme , the CLEC obtained from 0.2 ml of soluble enzyme (454 mg) was added. The enzyme amount which is capable of producing 1 micromole of dextrose per minute is defined as 1 enzyme unit.
5. **Determination of protein**: The determination of protein was carried out by Lowry's method, BSA was used as the standard and read at 660 nm(Lowry,O.H; Rosenbrough, N.J;Farr,A.L.and Randall,R.J. (1951), J.Biol.Chem,193,265-275.)
6. **Determination of reducing sugar and DE**; by Lane-Eynon titrimetric(Lane-Eynon, AOAC (1995), 16th ed., 44,pp-10) and DNS method(Miller.G.L.(1959), Anal.chem.31,426-428.). Percentage conversion was calculated as glucose obtained X 0.9. The term "D.E." (dextrose equivalent) refers to the reducing sugar content of a material, calculated as dextrose and expressed as percent of total solids.

### EXAMPLE 2

### Continuous hydrolysis of the starch to glucose by Glucoamylase CLEC

Continuous hydrolysis of the soluble starch and maltodextrin to glucose was carried out in a packed bed reactor. A jacketed glass column (114mmX 8mm) was used. 3.5 grams of the CLEC crystal was packed in the column which was held at 60°C. Continuous saccharification was carried out by passing ,4% and 10% (w/v) solution of starch (pH 4.5) and 10% maltodextrin (DE 12.5,pH 4.5) through the column at a dilution rate of 7-17 bed volumes per hour.

At 4% (W/V) soluble starch feed, a productivity of 55.13 g/L/h was obtained at a residence time of 5.4 minutes , when the concentration of the soluble starch was increased to 10%(W/V), the productivity increased to 110.58 g/L/h.at a residence time of 7.6 minutes When more suitable substrate for glucoamylase, maltodextrin of DE 12.5 , at a concentration of 10% (W/V) was used the productivity increased very much and a value of 463.7 g/L/h was obtained at residence time of 5.6 minutes . When the residence time was reduced to 3.4 minutes a productivity of 714.1 g/L/h was obtained.After 10h of continuous saccharification of 4% soluble starch solution, the activity of the CLEC was declined to 2.3 IU/gm from 5.1 IU/gm crystal(the half life).

### EXAMPLE 3

### Preparation of A Crosslinked HRP Crystal Formulation

A 50 g aliquot of Horse Radish Peroxidase(HRP) in powder form (Type 11, SIGMA) was purified by initial fractional precipitation by Ammonium sulphate, centrifuged and the pellet was dialysed, (all the operations were done at 4 degree C) and freeze dried to give a powder. A 30 g HRP powder was mixed 780 ml of phosphate buffer, 10 mM, pH 7.2. Isopropyl alcohol (20%) was added followed by the addition of Tween-20 (1%). Solid ammonium sulphate was slowly added to the solution over a period of 1 hour until crystallisation starts. More salt is added slowly to complete the crystallisation (total Amm.sulphate- 390 gm).Crystallization was then allowed to proceed for about 5-20 hours. The crystals were allowed to settle and soluble protein was removed using a peristalic pump with tygon tubing having a 10 ml pipette at its end.. The crystal solution was then crosslinked as follows.

Crystals were dissolved in minimum amount of Phosphate buffer, 10 mM, pH 7.2. 5% glutaraldehyde solution in phosphate buffer, 10 mM, pH 7.2 was added slowly to the enzyme solution. After 1 hour , crystals formed were separated by centrifugation (5000 rpm, 5 min, 4 degree C) and washed successively with 0.1 M acetate buffer, pH 4.5; 6M urea,2M NaCl, and finally with acetate buffer, pH 4.5. 30 gm crystals of rhombohedral shape was obtained.

A 10 gram aliquot of the above-prepared crosslinked HRP enzyme crystals was suspended in 100 ml storage buffer (10 mM Acteate ,pH 4.5) and the mixture poured into a sintered glass funnel (porosity .about.10-20.microns.) at room temperature. The buffer was then removed from the enzyme. The enzyme crystals were exposed to the surfactant AOT as described below. The buffer above the crosslinked HRP crystals was filtered in a sintered glass funnel (as described above), keeping the crosslinked enzyme crystals wet throughout the process. The surfactant was added together with the solvent 2-propanol, such that the ratio of surfactant:crosslinked enzyme crystals was 1:1 (6 g HRP:6 g surfactant=5.7 ml). This was done by pouring a mixture of 28.3 ml 2-propanol and 5.7 ml surfactant, for a total of 34 ml, on top of the crosslinked enzyme crystals. A gentle suction was applied to ensure that the crosslinked enzyme crystals were coated with the surfactant and so that the enzyme cake did not dry. After 30 minutes at room temperature, the mixture was then transferred to a lyophilizer and freeze dried to a water content of about 5-10%.

### EXAMPLE 4

### Preparation of a Crosslinked plant leaf peroxidase Crystal Formulation

A sltu-ry of 10 g or 25 ml of purified Ipomea (leaf) peroxidase (IPP) or Saccharum (Leaf) peroxidase (SPP) was mixed with Crystal seeds (0.27 g protein), and the mixture was maintained at a temperature 4degree. C. Crystallization was then allowed to proceed for a period of 1-2 days. The mother liquor was removed using a Buchner funnel with a 1.micron. filter. The crystal yield was about 10.2 grams. The crystal solution was then crosslinked as follows.
Crosslinking was carried out using 1.5 ml of 50% glutaraldehyde crosslinking agent per gram of enzyme. More particularly, a 15 ml aliquot of crosslinking agent was added to 10 g enzyme over a total addition time of 30 minutes to 1 hour. The mixture was allowed to mix for 4 hours at room temperature for crosslinking, keeping the pH at 4.5 at all times by Sodium acetate buffer, 10mM.. The crosslinking reaction was stopped by washing the crosslinked crystals. Then the crosslinked enzyme crystals were suspended in buffer (NaAc, 10 mM, pH 4.5).

A 2 gram aliquot of the above-prepared crosslinked PP enzyme crystals was suspended in 10 ml storage buffer (NaAc, 10 mM pH 4.5) and the mixture poured into a sintered glass funnel (porosity .about.10-20.mu.) at room temperature. The enzyme crystals were exposed to the surfactant AOT. The buffer above the crosslinked PP crystals was filtered in a sintered glass funnel (as described above), keeping the crosslinked enzyme crystals wet throughout the process. The surfactant was added together with the solvent isopropanol, such that the ratio of surfactant:crosslinked enzyme crystals was 1:1.5 (3 g PP:3 ml surfactant=5 ml). This was done by pouring a mixture of 2 ml isopropanol and 3 ml surfactant, for a total of 5 ml, on top of the crosslinked enzyme crystals. A gentle suction was applied to ensure that the crosslinked enzyme crystals were coated with the surfactant and so that the enzyme cake did not dry. After 30 minutes at room temperature, the mixture was then transferred to a freeze drier and dried to a moisture content of 2-10%. Lyophilized crosslinked enzyme crystal formulations prepared as described above may be stored at room temperature or at 4.degree. C., prior to their use in organic solvents.

### EXAMPLE 5

### Preparation of HRP CLEC:

The HRP was purified as in example 2.30 gm of the HRP was dissolved in 780 ml of Tris buffer, 10mM pH 8.7. to which 1,5 pentane diol (20%) and AOT (1%) was added along with solid ammo.sulphate (390 gms) to crystallize the enzyme. The crystals were allowed to form overnight.

The crystals were dissolved in minimum volume of 10 mM Tris buffer , pH 8.7. Cross linking agent was prepared by mixing glutaraldehyde with 4.5 ml of Tris buffer, 10 mM, pH 8.7, and the enzyme solution was added dropwise to the cross linking solution .Cross linking was carried out for 1 hour and the cross linked crystals centrifuged and washed with acetate buffer ,pH 4.5, 6M urea, 2M NaCl and finally with acetate buffer pH 4.5.

### EXAMPLE 6

30 gm of purified HRP is dissolved in 10mM phosphate buffer, pH 5.7, isoamyl alcohol (1%) and cetrimide (20%) were added seperately. Solid ammonium sulphate was added to the mixture to crystallize the enzyme. The crystallization was allowed to continue for 5 hours. The crystals are recovered by centrifugation. The crystals were dissolved in minimum volume of phosphate buffer, pH 5.7, 10mM. To this mixture 1% glutaraldehyde in phosphate buffer , 10mM, pH5.7, was added dropwise. Cross linking was carried out for 1 hour and the cross plinked crystals centrifuged and washed with acetate buffer, pH 4.5, 6M urea, 2M NaCl and finally with acetate buffer pH 4.5.

The peroxidase Enzyme activity was assayed by Spectrophotometric methods. The activity was assayed by the method of Bergmeyer (Bergmeyer H.U."Methods in Enzymatic Analysis"vol 1,Academic press,1974. Pp-457) in both pellet and supernatant. The following reaction mixture was employed: 0.004Mm ABTS, 0.002mM H₂O₂, 0.067M sodium phosphate buffer (pH-6), enzyme extract-100 (1; total volume- 2.4 ml. Enzyme activity evaluated by measuring change in optical density at 420 nm. The Volume activity was calculated from the change in absorbance per minute ΔE/ΔT and extinction coefficient for the system is (2.645x10³ mmol⁻¹ cm⁻¹),

### EXAMPLE 7

### Preparation of 2,4-dimethylphenol dimer by HRP CLEC:

0.122 grams of 2,6-dimethylphenol was dissolved in 6ml of isopropanol and 200mg Crosslinked crystals of Horseradish Peroxidase was added while stirring at 50 .degree. C. To the reaction mixture, add 30% hydrogen peroxide was added at a rate of 20µl per hour for 5 hours. Upon completion of the addition of the hydrogen peroxide, the isopropanol was removed by distillation. 0.06 grams of 2,6-dimethylphenol were added to the reaction mix and heated for 1.5 hours at 100.degree. C. The product was collected by filtration and washed twice with 300 ml of toluene at 30.degree. C. for 30 minutes. After drying, 0.08 gm. of 2,4-dimethylphenol dimer was obtained.

### The main advantage of the present invention are:

1. The cross-linked crystal matrix in a CLEC provides its own support. Expensive carrier beads, glasses, gels, or films are not required in order to tie down the enzyme catalyst, as they are in presently-available immobilization methods.
2. The concentration of enzyme in a CLEC is close to the theoretical packing limit that can be achieved for molecules of a given size, greatly exceeding densities achievable even in concentrated solutions. The entire CLEC consists of active enzyme (and not inactive carrier), and thus, the diffusion-related reduction of enzyme reaction rates usually observed with conventionally immobilized enzymes relative to enzymes in solution should be minimized, since the mean free path for substrate and product between active enzyme and free solvent will be greatly shortened for CLECs (compared to a conventional immobilized enzyme carrier particles).
3. These high protein densities will be particularly useful in biosensor, analytical and other applications requiring large amounts of protein in small volumes. In industrial processes, the superior performance and compactness of CLECs results in significant operating economies, by increasing the effective activity of a given volume of catalyst, thereby allowing reductions in plant size, as well as capital costs.
4. CLECs are relatively monodisperse, with a macroscopic size and shape reflecting natural crystal growth characteristics of the individual enzyme catalysts. Replacement of existing carrier-immobilized enzyme media with CLECs should not be difficult, since both systems are comparable in size and shape, and both can be similarly recovered from feedstock by any number of simple methods, including basic economical operations such as filtration, centrifugation, decantation of solvent, and others.
5. The use of lyophilized CLECs permits routine handling and storage of these materials prior to use (dry storage at room temperature without refrigeration, for extended periods of time). Lyophilized CLECs also allow for routine formulation by direct addition of solvents and substrates of interest, without lengthy solvent exchange processes, or the formation of amorphous suspensions. The lyophilized CLEC form extends the general utility of the enzymes as catalysts to a broader spectrum of enzymes and functional conditions.
6. The cross-linking of the crystallized enzyme stabilizes and strengthens the crystal lattice and the constituent enzyme molecules, both mechanically and chemically. As a result, a CLEC may be the only means of achieving significant concentrations of active enzyme catalyst in harsh aqueous, organic, near-anhydrous solvents, or in aqueous-organic solvent mixtures. The use of enzymes as catalysts in organic syntheses has been hampered by their tendency to denature in the presence of non-aqueous solvents, and particularly, in mixtures of aqueous and non-aqueous solvents.
7. In CLECs, the restriction of conformational mobility that leads to stability is provided by the inter-molecular contacts and cross-links between the constituent enzyme molecules making up the crystal lattice, rather than by the near-absence of water in the medium. As a result, intermediate water concentrations can be tolerated by enzymes when formulated as CLECs, as has previously not been possible. In commercial applications, aqueous-organic solvent mixtures allow manipulation of product formation by taking advantage of relative solubilities of products and substrates. Even in aqueous media, enzyme catalysts, immobilized or soluble, are subject to mechanical forces within a chemical reactor that can lead to denaturation and a shortened half-life. The chemical cross-links within the CLEC provide the necessary mechanical strength that results in increased reactor life for the enzyme catalyst.
8. The advantage of a CLEC is that as a result of its crystalline nature, a CLEC can achieve uniformity across the entire cross-linked crystal volume. Crystalline enzymes as described herein are grown and cross-linked in an aqueous environment and, therefore, the arrangement of molecules within the crystal lattice remains uniform and regular. This uniformity is maintained by the intermolecular contacts and chemical cross-links between the enzyme molecules constituting the crystal lattice, even when exchanged into other aqueous, organic or near-anhydrous media, or mixed aqueous/organic solvents. In all of these solvents, the enzyme molecules maintain a uniform distance from each other, forming well-defined stable pores within the CLECs that facilitate access of substrate to the enzyme catalysts, as well as removal of product. Uniformity of enzyme activity is critical in industrial, medical and analytical applications where reproducibility and consistency are paramount.
9. CLEC exhibits an increased operational and storage half-life. Lattice interactions, even in the absence of cross-linking, are known to stabilize proteins, due in part to restrictions of the conformational degrees of freedom needed for protein denaturation. In CLECs, the lattice interactions, when fixed by chemical cross-links, are particularly important in preventing denaturation, especially in mixtures of aqueous and non-aqueous solvents. Cross-linked immobilized enzyme crystals stored in anhydrous solvents will be even further protected from microbial contamination and damage, which is a serious problem in storing large quantities of protein in a nutrient rich, aqueous environment. In the case of a lyophilized CLEC, the immobilized enzyme is stored in the absence of solvent. That, and the stabilization achieved by cross-linking allows for the storage in the absence of refrigeration for long periods of time.
10. CLEC exhibits enhanced temperature stability as a consequence of the crosslinks stabilizing the crystal lattice. Carrying out reactions at a higher temperature than that used with conventional methods would increase reaction rates for the chemical reactions of interest, both thermodynamically, and by enhancing the diffusion rate into and out of the crystal lattice of CLECs. These combined effects would represent a major improvement in reaction efficiency, because they would maximize the productivity of a given quantity of enzyme catalyst, which is generally the most expensive component of the reaction process The temperature stability exhibited by CLECs is remarkable because most enzyme systems require mild reaction conditions. CLECs would also be stabilized against denaturation by transient high temperatures during storage.
11. In CLEC the pores of regular size and shape are created between individual enzyme molecules in the underlying crystal lattice. This restricted solvent accessibility greatly enhances the metal ion or cofactor retention characteristics of CLEC as compared to conventionally immobilized enzymes and enzymes in solution. This property of CLEC will permit the use of economically superior continuous-flow processes in situations where enzyme would .otherwise be inactivated by metal ion or cofactor leaching.
12. Glucoamylase CLEC and Peroxidase CLEC are versatile biocatalysts, which can be used for a variety of biotransformations and bioremediations in aqueous, biphasic and solvent media.

## Claims

1. A process for the preparation of stable cross-linked amylase or peroxidase crystals, wherein the amylase is a glucoamylase and the peroxidase is a plant peroxidase, the process comprising the steps of:
(a) crystallizing the amylase or peroxidase enzymes in an aqueous solution containing a suitable salt in the presence of an organic cosolvent at a temperature ranging between 4° and 10°C for a period ranging between 5 hours to 20 days to obtain enzyme crystals having a particle size ranging between 50 to 150 microns;
(b) reacting the enzyme crystals obtained in step (a) with a multifunctional cross-linking agent in the presence of buffer of a pH ranging between 3 - 10 at a temperature ranging between 4° to 10°C to get the cross-linked enzyme crystals, wherein said cross-linking agent is selected from the group consisting of glutaraldehyde and starch dialdehyde;
(c) washing the cross-linked crystals with a reagent that is capable of removing the excess of said multifunctional cross-linking agent so as to obtain the washed cross-linked enzyme; and
(d) coating the cross-linked enzyme crystals with a surfactant selected from the group consisting of cetyl trimethyl ammonium bromide, dioctyl sulfosuccinate, nonyl phenol ethoxylate, sorbitan trioleate, Tween 20, Tween 80 and Triton X-100 and lyophilizing it to obtain the stable product.

2. The process as claimed in claim 1 wherein said plant peroxidase is selected from the group consisting of Horseradish, Ipomea or Saccharum peroxidases.

3. The process as claimed in claim 1 or Claim 2, wherein said crystal is a microcrystal having a cross-section of 100 microns or less.

4. The process as claimed in any of claims 1 to 3 wherein said surfactant provides a weight ratio of crosslinked enzyme crystals to surfactant between about 1:1 and about 1:5, preferably between about 1:1 and about 1:2.

5. The process as claimed in any of claims 1 to 4, wherein the obtained lyophilized cross-linked enzyme crystal is having resistance to exogenous proteolysis, such that said crosslinked enzyme crystal retains at least 91% of its initial activity after incubation for three hours in the presence of a concentration of protease that causes the soluble uncrosslinked form of the enzyme that is crystallized to form said enzyme crystal that is crosslinked to loose at least 94% of its initial activity under the same conditions, wherein said crystal is in lyophilized form.

6. Cross-linked glucoamylase and plant peroxidase enzyme crystals obtainable according to the process as defined in any of claims 1 to 5.

7. Cross-linked peroxidase enzyme crystals as claimed in claim 6 wherein said plant peroxidase is selected from the group consisting of Horseradish, Ipomea or Saccharum peroxidases.

8. A process for the preparation of glucose by hydrolysis of starch or maltodextrin, said process comprising continuous saccharification of starch and/or maltodextrin by reacting a starch and/or maltodextrin solution in a packed bed reactor with cross-linked glucoamylase enzyme crystals as claimed in claim 6.

9. A process for the preparation of 2,4-dimethyl phenol dimer from monomer, said process comprising reacting 2,4-dimethyl phenol monomer with Horseradish peroxidase enzyme crystals as claimed in claim 7 in the presence of hydrogen peroxide.

## Patentansprüche

1. Verfahren zur Herstellung von stabilen vernetzten Amylase- oder Peroxidasekristallen, wobei die Amylase eine Glucoamylase ist und die Peroxidase eine Pflanzenperoxidase ist und wobei das Verfahren die folgenden Schritte umfasst:
(a) Kristallisieren der Amylase- oder Peroxidaseenzyme in einer wässrigen Lösung, die ein geeignetes Salz enthält, in der Anwesenheit eines organischen Colösungsmittels, bei einer Temperatur, die in einem Bereich von 4° und 10°C liegt, über einen Zeitraum, der in einem Bereich von 5 Stunden bis 20 Tagen Jiegt, um Enzymkristalle zu erhalten, die eine Partikelgröße haben, die in einem Bereich von 50 bis 150 µm liegt;
(b) Reagieren der in Schritt (a) erhaltenen Enzymkristalle mit einem multifunktionalen Vernetzungsmittel in der Anwesenheit eines Puffers mit einem pH, der in einem Bereich von 3 bis 10 liegt, bei einer Temperatur, die in einem Bereich von 4° bis 10°C liegt, um die vernetzten Enyzmkristalle zu erhalten, wobei das Vernetzungsmittel ausgewählt ist aus der Gruppe bestehend aus Glutaraldehyd und Stärkedialdehyd;
(c) Waschen der vernetzten Kristalle mit einem Reagenz, das in der Lage ist, den Überschuss des multifunktionalen Vernetzungsmittels zu entfernen, so dass das gewaschene vernetzte Enzym erhalten wird; und
(d) Beschicht en der vernetzten Enzymkristalle mit einem Tensid ausgewählt aus der Gruppe bestehend aus Cetyltrimethylammoniumbromid, Dioctylsulfosuccinat, Nonylphenolethoxylat, Sorbitantrioleat, Tween 20, Tween 80 und Triton X-100, und Lyophilisieren, um das stabile Produkt zu erhalten.

2. Verfahren nach Anspruch 1, wobei die Pflanzenperoxidase ausgewählt ist aus der Gruppe bestehend aus Meerrettich-, Prunkwinden- oder Zuckerrohrperoxidasen.

3. Verfahren nach Anspruch 1 oder Anspruch 2, wobei der Kristall ein Mikrokristall mit einem Querschnitt von 100 µm oder weniger ist.

4. Verfahren nach einem der Ansprüche 1 bis 3, wobei das Tensid ein Gewichtsverhältnis von vernetzten Enzymkristallen zu Tensid zwischen ungefähr 1:1 und ungefähr 1:5, bevorzugt zwischen ungefähr 1:1 und ungefähr 1:2, vorsieht.

5. Verfahren nach einem der Ansprüche 1 bis 4, wobei der erhaltene lyophilisierte vernetzte Enzymkristall eine Widerstandsfähigkeit gegenüber exogener Proteolyse besitzt, so dass der vernetzte Enzymkristall wenigstens 91 % seiner ursprünglichen Aktivität nach dreistündiger Inkubation in der Anwesenheit einer Proteasenkonzentration beibehält, die bei der löslichen nichtvernetzten Enzymform, die kristallisiert ist, um den Enzymkristall zu bilden, der vernetzt ist, dazu führt, unter den gleichen Bedingungen wenigstens 94% ihrer ursprünglichen Aktivität zu verlieren, wobei der Kristall in lyophilisierter Form vorliegt.

6. Vernetzte Glucoamylase- und Pflanzenperoxidase-Enzymkristalle, erhältlich gemäß dem Verfahren, das in einem der Ansprüche 1 bis 5 definiert ist.

7. Vernetzte Peroxidaseenzymkristalle nach Anspruch 6, wobei die Pflanzenperoxidase ausgewählt ist aus der Gruppe bestehend aus Meerrettich-Prunkwinden- oder Zuckerrohrperoxidasen.

8. Verfahren zur Herstellung von Glucose durch Hydrolyse von Stärke oder Maltodextrin, wobei das Verfahren kontinuierliche Verzuckerung von Stärke und/oder Maltodextrin durch Reagieren einer Stärke- und/oder Maltodextrinlösung mit vernetzten Glucoamylaseenzymkristallen nach Anspruch 6 in einem gepackten Bettreaktor umfasst.

9. Verfahren zur Herstellung von 2,4-Dimethylphenoldimer aus Monomer, wobei das Verfahren Reagieren von 2,4-Dimethylphenolmonomer mit Meerrettichperoxidaseenyzmkristallen nach Anspruch 7 in der Anwesenheit von Wasserstoffperoxid umfasst.

## Revendications

1. Procédé de préparation de cristaux d'amylase ou de peroxydase réticulés stables, dans lequel l'amylase est une glucoamylase et la peroxydase est une peroxydase végétale, le procédé comprenant les étapes consistant à :
(a) cristalliser les enzymes amylase ou peroxydase dans une solution aqueuse contenant un sel approprié en présence d'un co-solvant organique à une température située entre 4° et 10°C pendant une période allant de 5 heures à 20 jours pour obtenir des cristaux d'enzyme ayant une granulométrie allant de 50 à 150 microns ;
(b) faire réagir les cristaux d'enzyme obtenus à l'étape (a) avec un agent réticulant polyfonctionnel en présence d'un tampon ayant un pH allant de 3 à 10 à une température située entre 4° et 10°C pour obtenir les cristaux d'enzyme réticulés, dans lequel ledit agent réticulant est choisi dans le groupe constitué par le glutaraldéhyde et le dialdéhyde d'amidon ;
(c) laver les cristaux réticulés avec un réactif capable de retirer l'excédent dudit agent réticulant polyfonctionnel de manière à obtenir l'enzyme réticulée lavée ; et
(d) enrober les cristaux d'enzyme réticulés avec un tensioactif choisi dans le groupe constitué par le bromure de cétyltriméthylammonium, le dioctylsulfosuccinate, l'éthoxylate de nonylphénol, le trioléate de sorbitane, Tween 20, Tween 80 et Triton X-100 et les lyophiliser pour obtenir le produit stable.

2. Procédé selon la revendication 1, dans lequel ladite peroxydase végétale est choisie dans le groupe constitué par les peroxydases du raifort, de l'ipomée ou de la canne.

3. Procédé selon la revendication 1 ou 2, dans lequel ledit cristal est un microcristal ayant une section transversale de 100 microns ou moins.

4. Procédé selon l'une quelconque des revendications 1 à 3, dans lequel ledit tensioactif produit un rapport en poids entre les cristaux d'enzyme réticulés et le tensioactif situé entre environ 1/1 et environ 1/5, de préférence entre environ 1/1 et environ 1/2.

5. Procédé selon l'une quelconque des revendications 1 à 4, dans lequel le cristal d'enzyme réticulé lyophilisé obtenu a une résistance à la protéolyse exogène telle que ledit cristal d'enzyme réticulé conserve au moins 91 % de son activité initiale après incubation pendant trois heures en présence d'une concentration de protéase qui permet à la forme non réticulée soluble de l'enzyme qui est cristallisée de former ledit cristal d'enzyme qui est réticulé pour qu'il libère au moins 94 % de son activité initiale dans les mêmes conditions, ledit cristal étant sous forme lyophilisée.

6. Cristaux de l'enzyme glucoamylase ou peroxydase végétale réticulés, pouvant être obtenus selon le procédé défini dans l'une quelconque des revendications 1 à 5.

7. Cristaux de l'enzyme peroxydase réticulés selon la revendication 6, dans lesquels ladite peroxydase végétale est choisie dans le groupe constitué par les peroxydases du raifort, de l'ipomée ou de la canne.

8. Procédé de préparation du glucose par hydrolyse de l'amidon ou de la maltodextrine, ledit procédé comprenant la saccharification continue de l'amidon et/ou de la maltodextrine en faisant réagir une solution d'amidon et/ou de maltodextrine dans un réacteur à lit garni avec des cristaux de l'enzyme glucoamylase réticulés selon la revendication 6.

9. Procédé de préparation du dimère de 2,4-diméthylphénol à partir du monomère, ledit procédé comprenant la réaction du monomère de 2,4-diméthylphénol avec des cristaux de l'enzyme peroxydase du raifort selon la revendication 7 en présence du peroxyde d'hydrogène.
